# EUROPEAN PATENT APPLICATION

(11) **EP 4 529 938 A1**
(43) Date of publication of application: **02.04.2025**
(21) Application number: 23306602.6
(22) Date of filing: 26.09.2023
(51) Int. Cl.: A61M 5/315

(54) **SYRINGE PLUNGER ROD WITH VARIABLE DIAMETER THREADED CONNECTION**

(71) Applicant: Becton Dickinson France, 38800 Le Pont-de-Claix (FR)
(72) Inventor: EUVRARD, Nicolas, London SW17 0JF (GB)
(74) Representative: Germain Maureau

(57) **Abstract**

Provided herein is a syringe including a syringe barrel and a plunger assembly axially movable within a chamber of the syringe barrel. The plunger assembly includes a plunger rod having a threaded extension member at a distal end thereof and a stopper secured to the extension member. The stopper includes a main body that defines a threaded inner cavity at a proximal end thereof that mates with the threaded extension member, and a plurality of ribs extending around an outer circumference of the main body. The threaded extension member is configured to have an outer diameter that varies along a length thereof. The threaded extension member may be threaded into the threaded inner cavity to partially and fully threaded positions, to vary a location of an interference between the threaded extension member and the threaded inner cavity along an axial length of the stopper.

## Description

### BACKGROUND OF THE INVENTION

### Field of the Invention

The present disclosure relates generally to a plunger assembly for use with a syringe and, more particularly, to a plunger assembly that includes a plunger rod having a variable diameter threaded connection that mates with the stopper.

### Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper is typically made of elastomeric material and is adapted to ensure the container closure integrity (CCI) of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The tail portion of the stopper may include a cavity formed therein configured to receive a distal head of the plunger rod, so as to engage the plunger rod with the stopper. According to some embodiments, each of the cavity and the plunger head may be configured to provide a threaded engagement therebetween to secure the plunger rod to the stopper. The head portion of the stopper may include a plurality of annular, outwardly protruding ribs formed on an external cylindrical wall thereof, with the ribs applying a contact pressure against an inner surface of the syringe barrel to form a seal therewith that ensures the CCI of the syringe. A series of two or three ribs, for example, may be spaced apart longitudinally along the stopper main body, with one or more of the ribs positioned back near the tail portion, such that those rib(s) are formed on the stopper at a location that is axially/longitudinally aligned with where the cavity is formed in the stopper.

It is recognized that ensuring of the CCI of the syringe is vital during both transport/storage of the syringe and use of the syringe, but that the requirements and/or considerations for maintaining of the CCI can vary based on the particular lifecycle stage of the syringe. That is, it is recognized that - in order to maintain CCI - a higher contact pressure may need to be maintained between the stopper and the syringe barrel during transport/storage than during use. In particular, a higher contact pressure may need to be maintained during transport/storage between a proximal-most rib of the stopper and the syringe barrel in order to maintain CCI. This requirement to maintain a higher contact pressure at the proximal rib may be required during transport/storage of the syringe due to: (1) a high pressure differential across the proximal rib of the syringe stopper of 0-0.5 bar, for example, such as may occur during plane shipment when a pressure drop is experienced at a high altitude, and (2) the prolonged length of time to which the proximal rib of the syringe stopper is exposed to such a pressure differential.

This requirement to maintain a higher contact pressure at the stopper proximal rib is opposed to the contact pressure requirements during use of the syringe. That is, during use of the syringe, because of the user force applied onto the stopper, there is a high pressure differential across the stopper distal rib (i.e., high pressure inside the drug compartment vs atmospheric pressure outside) due to the injection of the drug through the syringe needle (Bernoulli and Hagen-Poiseuille equations). Typically, a pressure differential across the needle is required to compensate for the pressure drop due to the needle, with this pressure differential ranging from half a bar to several bar of pressure differential across the stopper (when the viscosity of the fluid is higher than that of water. However, during use there is no significant pressure differential across the proximal rib of the stopper. While a higher contact pressure between the stopper and the syringe barrel could be maintained at the proximal rib during use of the syringe, such high contact pressure is undesirable as it increases the friction between the stopper and inner surface of the syringe barrel as the stopper is moved through the syringe barrel (i.e., increases the gliding force), making the syringe less user friendly.

Accordingly, a need exists in the art for a plunger assembly design that is able to provide a higher contact pressure between the proximal rib of the stopper and the syringe barrel during transport/storage of the syringe and an acceptably low contact pressure between the stopper and the syringe barrel during use of the syringe. The plunger assembly would thus ensure the CCI of the syringe during both transport/storage and use, while also providing a lower gliding force when advancing the plunger assembly through the syringe barrel during use.

### SUMMARY OF THE INVENTION

Provided herein is a syringe including a syringe barrel having a barrel proximal end and a barrel distal end and defining a chamber configured for containing a fluid therein, and a plunger assembly axially movable within the chamber of the syringe barrel between a retracted position and an advanced position. The plunger assembly includes a plunger rod having a plunger rod proximal end and a plunger rod distal end, with a threaded extension member formed at the plunger rod distal end. The plunger assembly also includes a stopper secured to the plunger rod distal end. The stopper includes a main body defining a proximal end and a distal end, with the main body defining a threaded inner cavity at the proximal end configured to mate with the threaded plunger head to secure the stopper to the plunger rod. The stopper also includes a plurality of ribs extending from an outer surface of the main body and around an outer circumference of the main body, the plurality of ribs including at least a distal rib and a proximal rib. The threaded extension member is configured such that an outer diameter of the threaded extension member varies along a length thereof.

In certain configurations, the threaded extension member comprises a first threaded portion having a first outer diameter and a second threaded portion having a second outer diameter, with the first threaded portion distal from the second threaded portion and with the second outer diameter greater than the first outer diameter.

In certain configurations, the threaded extension member comprises a third threaded portion having a third outer diameter, wherein the third threaded portion is axially positioned between the first threaded portion and the second threaded portion, and wherein the third outer diameter is less than each of the second outer diameter and the first outer diameter.

In certain configurations, an inner diameter of the threaded inner cavity varies along a length thereof, with the inner threaded cavity comprising a proximal cavity portion and a distal cavity portion, wherein the distal cavity portion has a first inner diameter and the proximal cavity portion has a second inner diameter, with the second inner diameter greater than the first inner diameter.

In certain configurations, the threaded extension member is threadable into the threaded inner cavity to a partially threaded position and a fully threaded position, with the first threaded portion separated from a distal end of the threaded inner cavity when in the partially threaded position and the first threaded portion in abutment with the distal end of the threaded inner cavity when in the fully threaded position.

In certain configurations, with the threaded extension member in the partially threaded position, the second threaded portion is axially aligned with the proximal rib, with an interference present between the second threaded portion and the proximal cavity portion that applies a radially outward pressure to the proximal rib.

In certain configurations, with the threaded extension member in the partially threaded position, the first threaded portion and the third threaded portion are positioned distally from the proximal rib and aligned axially with an inter-rib region of the main body, between the proximal rib and the distal rib.

In certain configurations, with the threaded extension member in the fully threaded position, the second threaded portion and the third threaded portion are aligned axially with an inter-rib region of the main body, between the proximal rib and the distal rib, with an interference present between the second threaded portion and the inter-rib region that applies a radially outward pressure to the inter-rib region.

In certain configurations, with the threaded extension member in the fully threaded position, the first threaded portion is in abutment with the distal end of the threaded inner cavity, with an interference present between the first threaded portion and the distal cavity portion that applies a radially outward pressure to the main body.

In certain configurations, with the threaded extension member in either the partially threaded position or the fully threaded position, no interference is present between the third threaded portion and the inner cavity.

Also provided herein is a method of engaging a plunger rod with a stopper positioned within a syringe barrel, as part of assembly and use of a syringe. The method includes advancing the plunger rod into an inner volume of the syringe barrel and into contact with the stopper, wherein the stopper comprises a main body and at least a distal rib and a proximal rib extending from an outer surface of the main body and positioned circumferentially thereabout, with the main body defining a threaded inner cavity, and wherein the plunger rod comprises a threaded extension member at a distal end thereof, with the threaded extension member having an outer diameter that varies along a length thereof. The method also includes engaging the threaded extension member with the threaded inner cavity. In engaging the threaded extension member with the threaded inner cavity, the method further comprises threading the threaded extension member into the threaded inner cavity to a partially threaded position, where a distal end of the threaded extension member is separated from a distal end of the threaded inner cavity, and threading the threaded extension member into the threaded inner cavity to a fully threaded position, where the distal end of the threaded extension member is in abutment with the distal end of the threaded inner cavity. With the threaded extension member at the partially threaded position, an interference is present between the threaded extension member and the threaded inner cavity that applies a radially outward pressure to the proximal rib. With the threaded extension member at the fully threaded position, an interference is present between the threaded extension member and the threaded inner cavity that applies a radially outward pressure to the distal rib.

In certain configurations, the partially threaded position is a transportation and storage position of the syringe, where a radial force between the stopper and the syringe barrel prevents movement of the stopper within the syringe barrel, the radial force provided by a radial pressure applied by the distal rib against the syringe barrel and a radial pressure applied by the proximal rib against the syringe barrel, with the radial pressure applied by the proximal rib resulting from the interference present between the threaded extension member and the threaded inner cavity.

In certain configurations, the fully threaded position is a use position of the syringe, where a radial force between the stopper and the syringe barrel is maintained at an acceptable level that enables the stopper to be advanced distally through the syringe barrel.

In certain configurations, an inner diameter of the threaded inner cavity varies along a length thereof, with the inner threaded cavity comprising a proximal cavity portion and a distal cavity portion, the distal cavity portion having a first inner diameter and the proximal cavity portion has a second inner diameter, with the second inner diameter greater than the first inner diameter, and wherein the threaded extension member comprises a first threaded portion having a first outer diameter and a second threaded portion having a second outer diameter, with the first threaded portion distal from the second threaded portion and with the second outer diameter greater than the first outer diameter.

In certain configurations, with the threaded extension member in the partially threaded position, the second threaded portion is axially aligned with the proximal rib, with an interference present between the second threaded portion and the proximal cavity portion that applies a radially outward pressure to the proximal rib, and with the threaded extension member in the fully threaded position, the second threaded portion is positioned in an inter-rib region between the proximal rib and the distal rib, with an interference present between the second threaded portion and the inter-rib region that applies a radially outward pressure to the inter-rib region.

### BRIEF DESCRIPTION OF THE DRAWINGS

FIG. 1 is a perspective view of a syringe, with which embodiments of the disclosure may be implemented;
FIG. 2 is an exploded view of the syringe of FIG. 1;
FIG. 3 is a side view of the stopper included in the syringe of FIG. 1, according to a non-limiting embodiment described herein;
FIG. 4 is a side cross-sectional view of the stopper of FIG. 3, according to a non-limiting embodiment described herein;
FIG. 5 is a side view of a threaded extension member of the plunger rod included in the syringe of FIG. 1, according to a non-limiting embodiment described herein;
FIG. 6 is a side cross-sectional view of the mated stopper and threaded extension member, with the threaded extension member in a partially threaded position, taken along line 7-7 of FIG. 1, according to a non-limiting embodiment described herein; and
FIG. 7 is a side cross-sectional view of the mated stopper and threaded extension member, with the threaded extension member in a fully threaded position, taken along line 7-7 of FIG. 1, according to a non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

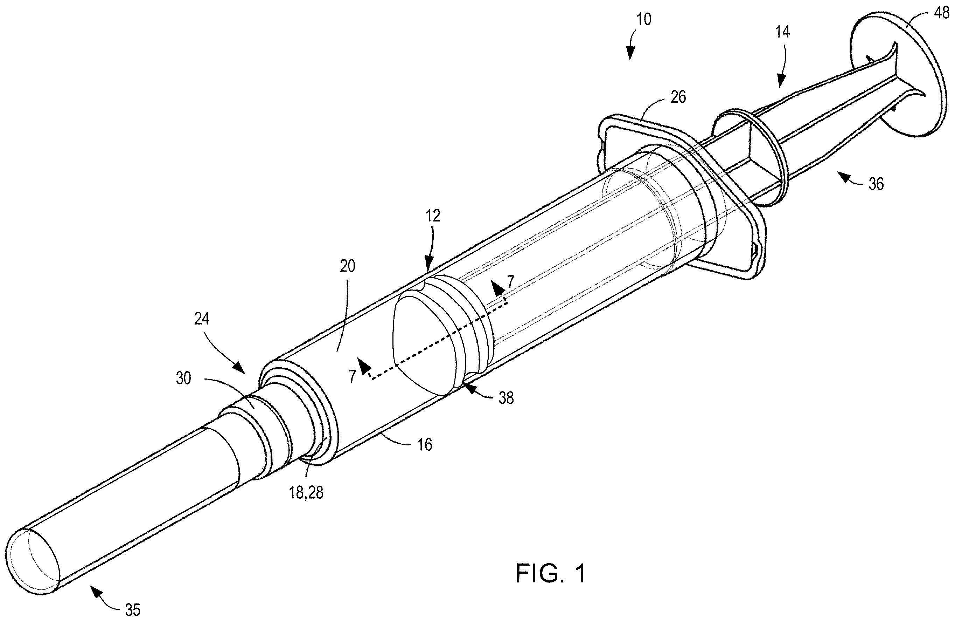

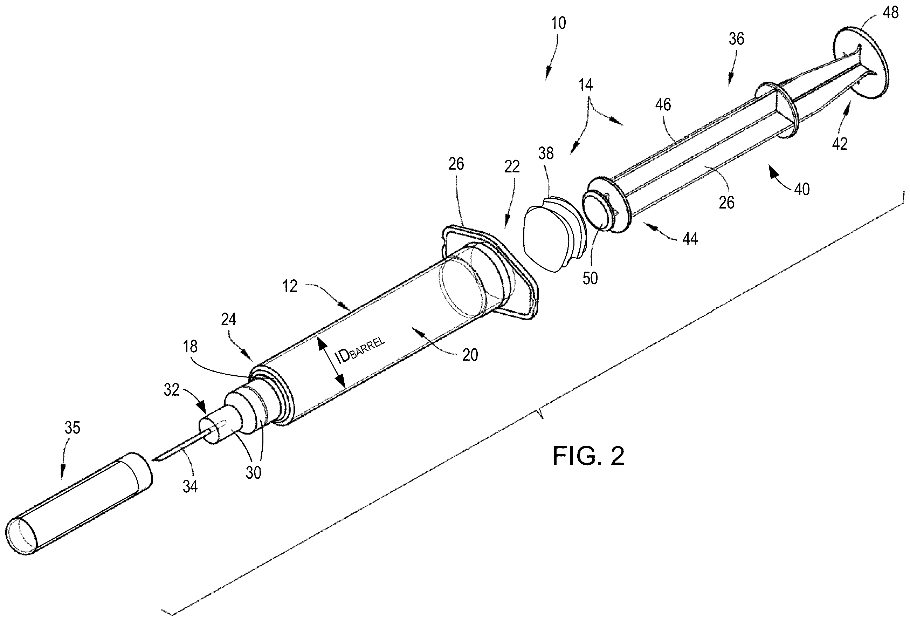

Referring to FIGS. 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical wall 16 and an end member 18 that collectively define a chamber 20 for retaining fluid therein, with the syringe barrel 12 formed of glass or plastic, according to various embodiments. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 and a plunger head or stopper 38. The plunger rod 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger rod 36 with respect to the syringe barrel 12. The plunger distal end 44 includes a threaded extension member 50 that extends out and is configured to mate with the stopper 38. As explained in further detail below, the threaded extension member 50 is configured to have a varied diameter at different locations along a length thereof, so as to provide for different mating configurations of the threaded extension member 50 with the stopper 38.

The stopper 38 of plunger assembly 14 is positioned at the plunger distal end 44 so as to be movable along with the plunger rod 36 within the chamber 20 of syringe barrel 12. The stopper 38 may be made from a material that is different from the material of the plunger rod 36 and that is capable of forming a tight seal with the syringe barrel 12 as it is advanced therethrough. In some embodiments, the stopper 38 is formed of an elastomeric material such as butyl rubber, styrene butadiene rubber, poly isoprene rubber, liquid silicone rubber, as non-limiting examples. In other embodiments, the stopper 38 may instead be formed of a polymeric material, such as cross-linked or thermoplastic elastomers, as non-limiting examples.

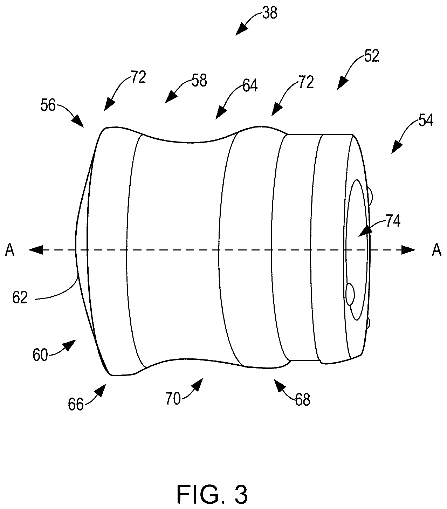

Referring now to FIGS. 3-6, and with continued reference to FIGS. 1 and 2, the structure of stopper 38 and extension member 50 of plunger rod 36 are shown in greater detail, according to an aspect or embodiment of the disclosure. The stopper 38 is defined by a main body 52 that includes an open proximal end 54 that engages with the distal end of plunger rod 36 and a closed distal end 56 configured to engage with the barrel 12 of syringe 10. The closed distal end 56 of the main body 52 includes a generally cylindrical portion 58 and a roof portion 60 that extends distally from cylindrical portion 58. In one embodiment, the roof portion 60 is configured as a conical portion that extends distally from cylindrical portion 58 to a tip 62 to provide the closed distal end 56. However, it is recognized that roof portion 60 may have other suitable shapes, such as being formed as a flat surface or domed surface, as other non-limiting examples.

An outer surface 64 of the main body 52 includes a plurality of ribs 66, 68 formed thereon that extend circumferentially around the entire outer surface 64. In the illustrated embodiment, the plurality of ribs 66, 68 includes a first rib 66 and a second rib 68, although it is recognized that the stopper 38 could include a greater number of ribs, such as three or four ribs, as explained further below. The first and second ribs 66, 68 are spaced apart longitudinally, such that the first rib 66 is positioned toward the proximal end 54 of the main body 52 and the second rib 68 is positioned toward the distal end 56 of the main body 52, with an inter-rib region 70 present between the first and second ribs 66, 68. Ribs 66, 68 are thus referred to as "distal rib 66" and "proximal rib 68."

According to embodiments of the disclosure, the structure of each of distal rib 66 and proximal rib 68 may be controlled to achieve a desired interaction between the stopper 38 and the barrel 12. According to one aspect of ribs 66, 68, the ribs 66, 68 extend radially outward a desired distance from the outer surface 64 (i.e., a rib thickness) to act as bearing points against the inner surface of the syringe barrel 12.

According to some aspects of the disclosure, the stopper 38 may further include one or more outer coatings or layers (not shown) applied to portions thereof, such as on a sealant or gliding surface thereof - i.e., on a contact surface 72 of distal rib 66 and proximal rib 68. The coating may be configured to reduce friction between the stopper 38 and barrel 12 when the stopper 38 is moved within the syringe 10. According to non-limiting embodiments, the coating may be provided as silicone oil, PTFE, ETFE, liquid silicone rubber, or another suitable coating that reduces friction.

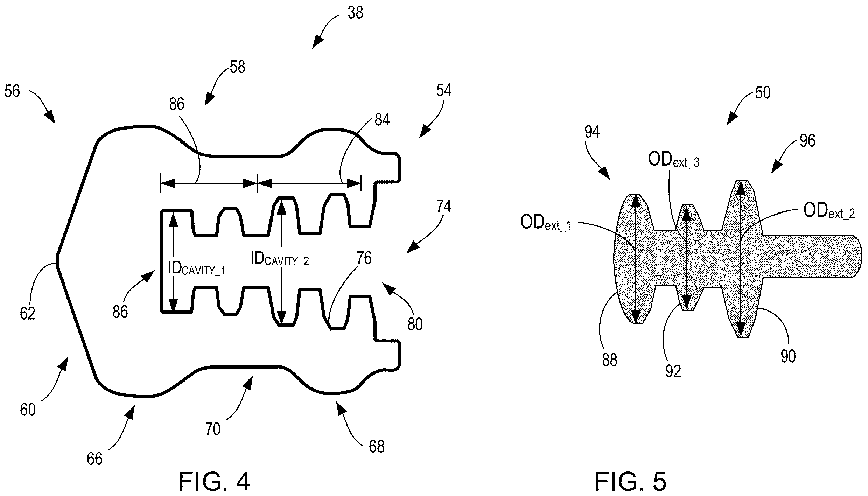

As shown in FIG. 4, the main body 52 of the stopper 38 is substantially hollow and is sized and configured to receive the extension member 50 of plunger rod 36 therein. The main body 52 of the stopper 38 defines an inner cavity 74 that is generally defined between a majority of the open proximal end 54 and the closed distal end 56 of the main body 52. With the inner cavity 74 formed as such, the proximal rib 68 is thus positioned at a location that is axially aligned (along longitudinal axis A) with a portion of the inner cavity 74. In some embodiments, the first rib 68 is also positioned at a location that is at least partially axially aligned with a portion of the inner cavity 74. The inner cavity 74 includes a threaded inner surface 76 that is configured to receive and mate with the threaded extension member 50 of plunger rod 36, with the threaded inner surface 76 including a cylindrical thread or a conical thread thereon, as non-limiting examples.

According to aspects of the disclosure, the stopper 38 and the plunger rod 36 are specifically configured to ensure that container closure integrity (CCI) of the syringe 10 is maintained during both transport/storage and use of the syringe 10, while also providing a lower gliding force when advancing the plunger assembly 14 through the syringe barrel 12 during use of the syringe 10. The threaded extension member 50 may be selectively engaged with the threaded inner cavity 74 at multiple distinct positions to vary an amount/location of a radially outward pressure applied from the stopper 38 against the syringe barrel 12, in order to maintain CCI and control a gliding force needed to advance the plunger assembly 14 during use of the syringe 10.

Referring first to FIG. 4, the threaded inner cavity 74 may be characterized as including a proximal end 80 and a distal end 82, with a proximal cavity portion 84 provided toward the proximal end 80 and a distal cavity portion 86 provided toward the distal end 82. The proximal cavity portion 84 and the distal cavity portion 86 of the threaded inner cavity 74 are configured to have different (inner) diameters, with the distal cavity portion 86 having a first inner diameter, ID_cavity _1, and the proximal cavity portion 84 having a second inner diameter, ID_cavity_2. As can be seen in FIG. 4, the second inner diameter, ID_cavity_2, of the proximal cavity portion 84 is greater than the first inner diameter, ID_cavity_1, of the distal cavity portion 86.

Referring now to FIG. 5, the threaded extension member 50 may be characterized as including a first threaded portion 88, a second threaded portion 90, and a third threaded portion 92. The threaded extension member 50 is configured such that the first threaded portion 88 is provided at a distal end 94 of threaded extension member 50, the second threaded portion 90 is provided at a proximal end 96 of threaded extension member 50, and the third threaded portion 92 is provided at a location axially between the first threaded portion 88 and the second threaded portion 90. The first, second, and third threaded portions 88, 90, 92 of the threaded extension member 50 are configured to have different (outer) diameters, with the first threaded portion 88 having a first outer diameter, OD_extension_1, the second threaded portion 90 having a second outer diameter, OD_ extension _2, and the third threaded portion 92 having a third outer diameter, OD_ extension _3. As can be seen in FIG. 5, the second outer diameter, OD_ extension _2, of the second threaded portion 90 is greater than the first outer diameter, OD_extension_1, of the first threaded portion 88, while the third outer diameter, OD_extension_3, of the third threaded portion 92 is less than both the first outer diameter, OD_extension_1, and the second outer diameter, OD_ extension _2.

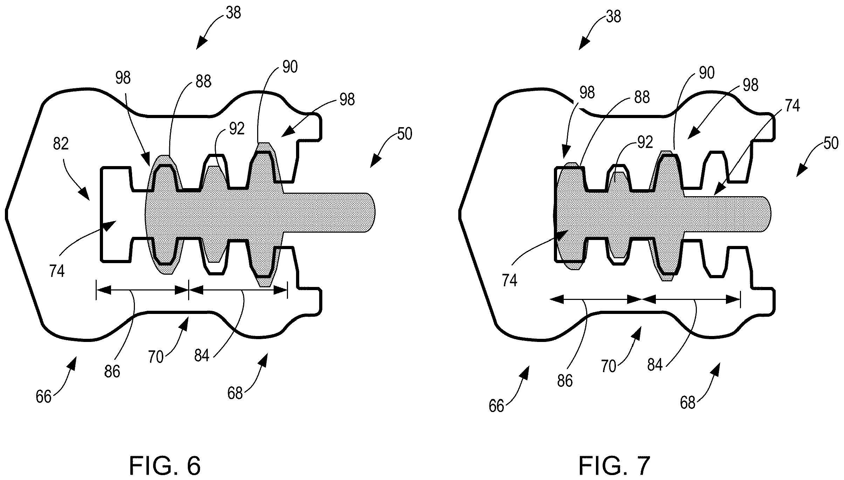

According to aspects of the disclosure, the inner cavity 74 and the extension member 50 are configured such that an interference (indicated by "98" in FIGS. 6 and 7) is present between the threaded extension member 50 and the threaded inner cavity 74 when mated together. According to embodiments, the interference 98 between the threaded extension member 50 and the threaded inner cavity 74 may be determined by the outer diameters of the threaded extension member 50 and the inner diameters of the threaded inner cavity 74 - i.e., outer diameters of threaded portion 88, 90, 92 and inner diameters of cavity portions 84, 86 - with it recognized that the amount of interference may vary based on the syringe design (e.g., dimensions, materials, etc.), but will always be greater than 0%. The interference 98 between the threaded extension member 50 and the threaded inner cavity 74 serves to apply a radially outward pressure to selective portion(s) of the stopper 38, such as to the distal rib 66, to an area of the main body 52 (e.g., inter-rib region 70), or to the proximal rib 68, based on an axial position of the extension member 50 within inner cavity 74. This radially outward pressure applied to portions of the stopper 38 functions (at least in part) to control the contact pressure and contact surface between the stopper 38 and the inner surface of syringe barrel 12, so as to help maintain CCI in the syringe 10 and/or determine the gliding force when advancing the plunger assembly 14 within the syringe barrel 12.

As shown in FIGS. 6 and 7, with the threaded extension member 50 mated with the threaded inner cavity 74, an interference may be present between the second threaded portion 90 and the proximal cavity portion 84, due to the second outer diameter, OD_ extension _2, of second threaded portion 90 being larger than the second inner diameter, ID_cavity_2, of the proximal cavity portion 84. Additionally, an interference may be present between the first threaded portion 88 and the distal cavity portion 86, due to the first outer diameter, OD_ extension _1, of first threaded portion 88 being larger than the first inner diameter, ID_cavity_1, of the distal cavity portion 86. In some embodiments, the third threaded portion 92 is sized (i.e., with third outer diameter, OD_ extension _3) such that no interference is present between the third threaded portion 92 and either of the proximal or distal cavity portions 84, 86 of the threaded inner cavity 74.

As shown in FIGS. 6 and 7, according to aspects of the disclosure, the threaded extension member 50 is threadable into the threaded inner cavity 74 to distinct positions that include a partially threaded position (FIG. 6) and a fully threaded position (FIG. 7). In the partially threaded position, the threaded extension member 50 is positioned within the threaded inner cavity 74 such that the first threaded portion 88 is separated from the distal end 82 of the threaded inner cavity 74. In the fully threaded position, the threaded extension member 50 is positioned within the threaded inner cavity 74 such that the distal end 94 of first threaded portion 88 is in abutment with the distal end 82 of the threaded inner cavity 74. By selectively threading the threaded extension member 50 into the threaded inner cavity 74 to the partially threaded position or the fully threaded position, a location and/or amount of radially outward pressure applied by the threaded extension member 50 against the stopper 38 - as determined by the location and amount of interference between the threaded extension member 50 against the threaded inner cavity 74 of stopper 38 - can also be controlled.

Referring to FIG. 6, with the threaded extension member 50 mated with the threaded inner cavity 74 in the partially threaded position, the second threaded portion 90 is engaged with proximal cavity portion 84 at a location that is axially aligned with the proximal rib 68. Accordingly, due to the interference between the second threaded portion 90 and the proximal cavity portion 84 (as the second outer diameter, OD_ extension _2, of second threaded portion 90 is larger than the second inner diameter, ID_cavity_2, of the proximal cavity portion 84), a radially outward pressure is applied to the proximal rib 68 - with this radially outward pressure increasing the contact pressure between the proximal rib 68 and the inner surface of syringe barrel 12.

Also, with the threaded extension member 50 mated with the threaded inner cavity 74 in the partially threaded position, the first threaded portion 88 is engaged with distal cavity portion 86 at a location that is axially aligned with the inter-rib region 70. Accordingly, due to the interference between the first threaded portion 88 and the distal cavity portion 86 (as the first outer diameter, OD_ extension _1, of first threaded portion 88 is larger than the first inner diameter, ID_cavity_1, of the distal cavity portion 86), a radially outward pressure is applied to inter-rib region 70 - with it recognized that this radially outward pressure does not impact the contact pressure between the stopper 38 and the inner surface of syringe barrel 12.

With the increased contact pressure between the proximal rib 68 and the inner surface of syringe barrel 12 provided by positioning of the threaded extension member 50 in the partially threaded position, along with an increased contact length between the stopper 38 and syringe barrel 12, it is recognized that positioning of the threaded extension member 50 in the partially threaded position provides strong protection against contamination ingress into the syringe 10 (especially at the location of proximal rib 68) and decreases the likelihood of stopper movement within the syringe barrel 12. Accordingly, positioning of the threaded extension member 50 in the partially threaded position may be desirable during transport and storage of the syringe, due to the substantial length of time the syringe may be in transport/storage (e.g., weeks or months) and/or due to potential pressure variations to which the syringe 10 may be exposed (e.g., a potential negative pressure differential during plane shipment) that may otherwise cause the stopper 38 to move within syringe barrel 12.

Referring to FIG. 7, with the threaded extension member 50 mated with the threaded inner cavity 74 in the fully threaded position, the second threaded portion 90 is engaged with proximal cavity portion 84 at a location that is axially distal from the proximal rib 68 and aligned with inter-rib region 70. Accordingly, due to the interference between the second threaded portion 90 and the proximal cavity portion 84 (as the second outer diameter, OD_ extension _2, of second threaded portion 90 is larger than the second inner diameter, ID_cavity_2, of the proximal cavity portion 84), a radially outward pressure is applied to the inter-rib region 70 - with it recognized that this radially outward pressure does not impact the contact pressure between the stopper 38 and the inner surface of syringe barrel 12. Also, as compared to the partially threaded position, no radially outward pressure is applied to the proximal rib 68, as the interference between the second threaded portion 90 and the proximal cavity portion 84 does not occur at a location axially aligned with the proximal rib 68.

Also, with the threaded extension member 50 mated with the threaded inner cavity 74 in the fully threaded position, the first threaded portion 88 is engaged with distal cavity portion 86 at a location that is axially aligned (at least partially) with the distal rib 66. Accordingly, due to the interference between the first threaded portion 88 and the distal cavity portion 86 (as the first outer diameter, OD_ extension _1, of first threaded portion 88 is larger than the first inner diameter, ID_cavity_1, of the distal cavity portion 86), a radially outward pressure is applied to distal rib 66 - with this radially outward pressure increasing the contact pressure between the distal rib 66 and the inner surface of syringe barrel 12.

With there being no increased contact pressure between the proximal rib 68 and the inner surface of syringe barrel 12 with the threaded extension member 50 in the fully threaded position, but only a desirable contact pressure between the distal rib 66 and the inner surface of syringe barrel 12 and a decreased contact length between the stopper 38 and syringe barrel 12, it is recognized that positioning of the threaded extension member 50 in the fully threaded position increases leak protection in the syringe 10 while maintaining an acceptably low gliding force when advancing the stopper 38 through syringe barrel 12 during use of the syringe 10. Accordingly, positioning of the threaded extension member 50 in the fully threaded position may be acceptable/beneficial during use of the syringe 10, due to the short duration of use and there being lower/no pressure variations to which the syringe 10 is exposed (i.e., little to no pressure variation/differential at the location of proximal rib 68).

With the construction of the threaded extension member 50 and the threaded inner cavity 74 as described above, a method for assembling and using a syringe may be provided, according to another aspect of the disclosure. The method provides for a selective mating of a plunger rod threaded extension member 50 with a threaded inner cavity 74 of stopper - in distinct partially threaded and fully threaded positions - so as to vary an amount/location of an interference therebetween, thereby controlling a corresponding radially outward pressure applied from the stopper 38 against the syringe barrel 12, in order to maintain CCI in the syringe 10 during transport/storage and use, and also controlling a gliding force needed to advance the plunger assembly 14 during use of the syringe 10.

In an initial step of assembly, the stopper 38 is inserted into the syringe barrel 12 (such as after pre-filling of the syringe barrel 12 with a medicament) and positioned at a desired location therein. The inserting/positioning of stopper 38 within syringe barrel 12 may be performed by a known process, such as vent tube stoppering for example. The vent tube stoppering may be performed via use of a stoppering rod, as known in the art, and prior to engagement of plunger rod 36 with stopper 38. Accordingly, the stopper 38 may be positioned within syringe barrel 12 with a low contact pressure and low/reduced contact length at both the distal and proximal ribs 66, 68, such that only a low stoppering force is needed - this being because the plunger rod 36 is not yet inserted into the stopper 38, such that there is no interference between the plunger rod 36 and stopper cavity at either distal or proximal rib regions.

After positioning of the stopper 38 within syringe barrel 12 at a desired location, the plunger rod may 36 then be advanced into the syringe barrel 12 and brought into contact with the stopper 38. The threaded extension member 50 may then be screwed into the threaded inner cavity 74 until reaching the partially threaded position. As described above, with the threaded extension member 50 at the partially threaded position, the contact pressure between the proximal rib 68 and the inner surface of syringe barrel 12 is increased (along with a potential increased contact length between the stopper 38 and syringe barrel 12), based on the second threaded portion 90 being axially aligned with the proximal rib 68 and the interference therebetween applying a radially outward pressure to the proximal rib 68. Accordingly, positioning of the threaded extension member 50 at the partially threaded position provides strong protection against contamination ingress into the syringe 10 and decreases the likelihood of stopper movement within the syringe barrel 12, during a subsequent transport and storage of the syringe 10 that occurs after initial assembly.

When it is desired to use the syringe 10 to administer the medicament therein to a patient, the threaded extension member 50 may then be further screwed into the threaded inner cavity 74 until reaching the fully threaded position (where the threaded extension member 50 abuts the distal end 82 of cavity 74). As described above, with the threaded extension member 50 at the fully threaded position, the contact pressure between the proximal rib 68 and the inner surface of syringe barrel 12 is decreased as compared to the partially threaded position (along with a decreased contact length between the stopper 38 and syringe barrel 12), based on the second threaded portion 90 being positioned distally from the proximal rib 68 and an interference between the second threaded portion 90 and the proximal cavity portion 84 now being aligned with the inter-rib region (with no/little interference at the location of proximal rib 68). Additionally, a desired contact pressure between the distal rib 66 and the inner surface of syringe barrel 12 may be provided, based on the first threaded portion 88 being a least partially axially aligned with the distal rib 66 and the interference therebetween applying a radially outward pressure to the distal rib 66. Accordingly, positioning of the threaded extension member 50 at the fully threaded position increases leak protection in the syringe 10 while not increasing a gliding force when advancing the stopper 38 through syringe barrel 12 during use of the syringe 10.

Beneficially, embodiments of the invention thus are directed to a syringe having a plunger assembly and stopper designed to ensure that CCI of the syringe is maintained during both transport/storage and use of the syringe, while also providing an acceptably low gliding force when advancing the plunger assembly through the syringe barrel during stoppering and use of the syringe. Each of a threaded extension member of the plunger rod and a threaded inner cavity of the stopper has a variable diameter along the length thereof that provides varying amounts/locations of interference between the threaded extension member and the threaded inner cavity when engaged. The threaded extension member may be selectively engaged with the threaded inner cavity at multiple distinct positions to vary an amount/location of the interference and a corresponding radially outward pressure applied from the stopper against the syringe barrel, in order to maintain CCI and control a gliding force needed to advance the plunger assembly during use of the syringe.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A syringe comprising:
a syringe barrel having a barrel proximal end and a barrel distal end and defining a chamber configured for containing a fluid therein; and
a plunger assembly axially movable within the chamber of the syringe barrel between a retracted position and an advanced position, the plunger assembly including:
a plunger rod having a plunger rod proximal end and a plunger rod distal end, with a threaded extension member formed at the plunger rod distal end; and
a stopper secured to the plunger rod distal end, the stopper including:
a main body defining a proximal end and a distal end, with the main body defining a threaded inner cavity at the proximal end configured to mate with the threaded extension member to secure the stopper to the plunger rod; and
a plurality of ribs extending from an outer surface of the main body and around an outer circumference of the main body, the plurality of ribs including at least a distal rib and a proximal rib;
**characterized in that** an outer diameter of the threaded extension member varies along a length thereof.

2. The syringe of claim 1, wherein the threaded extension member comprises a first threaded portion having a first outer diameter and a second threaded portion having a second outer diameter, with the first threaded portion distal from the second threaded portion and with the second outer diameter greater than the first outer diameter.

3. The syringe of claim 2, wherein the threaded extension member comprises a third threaded portion having a third outer diameter, wherein the third threaded portion is axially positioned between the first threaded portion and the second threaded portion, and wherein the third outer diameter is less than each of the second outer diameter and the first outer diameter.

4. The syringe of claim 2 or claim 3, wherein an inner diameter of the threaded inner cavity varies along a length thereof, with the inner threaded cavity comprising a proximal cavity portion and a distal cavity portion; and
wherein the distal cavity portion has a first inner diameter and the proximal cavity portion has a second inner diameter, with the second inner diameter greater than the first inner diameter.

5. The syringe of claim 4, wherein the threaded extension member is threadable into the threaded inner cavity to a partially threaded position and a fully threaded position, with the first threaded portion separated from a distal end of the threaded inner cavity when in the partially threaded position and the first threaded portion in abutment with the distal end of the threaded inner cavity when in the fully threaded position.

6. The syringe of claim 5, wherein with the threaded extension member in the partially threaded position, the second threaded portion is axially aligned with the proximal rib, with an interference present between the second threaded portion and the proximal cavity portion that applies a radially outward pressure to the proximal rib.

7. The syringe of claim 5, wherein with the threaded extension member in the partially threaded position, the first threaded portion and the third threaded portion are positioned distally from the proximal rib and aligned axially with an inter-rib region of the main body, between the proximal rib and the distal rib.

8. The syringe of any of claims 4-7, wherein with the threaded extension member in the fully threaded position, the second threaded portion and the third threaded portion are aligned axially with an inter-rib region of the main body, between the proximal rib and the distal rib, with an interference present between the second threaded portion and the inter-rib region that applies a radially outward pressure to the inter-rib region.

9. The syringe of claim 7 or claim 8, wherein with the threaded extension member in the fully threaded position, the first threaded portion is in abutment with the distal end of the threaded inner cavity, with an interference present between the first threaded portion and the distal cavity portion that applies a radially outward pressure to the distal rib.

10. The syringe of any of claims 7-9, wherein with the threaded extension member in either the partially threaded position or the fully threaded position, no interference is present between the third threaded portion and the inner cavity.

11. A method of engaging a plunger rod with a stopper positioned within a syringe barrel, as part of assembly and use of a syringe, the method comprising:
advancing the plunger rod into an inner volume of the syringe barrel and into contact with the stopper, wherein the stopper comprises a main body and at least a distal rib and a proximal rib extending from an outer surface of the main body and positioned circumferentially thereabout, with the main body defining a threaded inner cavity, and wherein the plunger rod comprises a threaded extension member at a distal end thereof, with the threaded extension member having an outer diameter that varies along a length thereof; and
engaging the threaded extension member with the threaded inner cavity;
wherein engaging the threaded extension member with the threaded inner cavity comprises:
threading the threaded extension member into the threaded inner cavity to a partially threaded position, where a distal end of the threaded extension member is separated from a distal end of the threaded inner cavity; and
threading the threaded extension member into the threaded inner cavity to a fully threaded position, where the distal end of the threaded extension member is in abutment with the distal end of the threaded inner cavity;
wherein, with the threaded extension member at the partially threaded position, an interference is present between the threaded extension member and the threaded inner cavity that applies a radially outward pressure to the proximal rib; and
wherein, with the threaded extension member at the fully threaded position, an interference is present between the threaded extension member and the threaded inner cavity that applies a radially outward pressure to the distal rib.

12. The method of claim 11, wherein the partially threaded position is a transportation and storage position of the syringe, where a radial force between the stopper and the syringe barrel prevents movement of the stopper within the syringe barrel, the radial force provided by a radial pressure applied by the distal rib against the syringe barrel and a radial pressure applied by the proximal rib against the syringe barrel, with the radial pressure applied by the proximal rib resulting from the interference present between the threaded extension member and the threaded inner cavity.

13. The method of claim 11 or claim 12, wherein the fully threaded position is a use position of the syringe, where a radial force between the stopper and the syringe barrel is maintained at an acceptable level that enables the stopper to be advanced distally through the syringe barrel.

14. The method of any of claims 11-13, wherein an inner diameter of the threaded inner cavity varies along a length thereof, with the inner threaded cavity comprising a proximal cavity portion and a distal cavity portion, the distal cavity portion having a first inner diameter and the proximal cavity portion has a second inner diameter, with the second inner diameter greater than the first inner diameter, and wherein the threaded extension member comprises a first threaded portion having a first outer diameter and a second threaded portion having a second outer diameter, with the first threaded portion distal from the second threaded portion and with the second outer diameter greater than the first outer diameter.

15. The method of claim 14, wherein with the threaded extension member in the partially threaded position, the second threaded portion is axially aligned with the proximal rib, with an interference present between the second threaded portion and the proximal cavity portion that applies a radially outward pressure to the proximal rib; and
wherein with the threaded extension member in the fully threaded position, the second threaded portion is positioned in an inter-rib region between the proximal rib and the distal rib, with an interference present between the second threaded portion and the inter-rib region that applies a radially outward pressure to the inter-rib region.
